Europäisches Patentamt

European Patent Office

Office européen des brevets

(11) Publication number: **0 241 134 B1**

# EUROPEAN PATENT SPECIFICATION

(45) Date of publication of patent specification: **30.10.91**

(51) Int. Cl.⁵: **A61K 31/535**, //(A61K31/535, 31:13,31:435,37:24)

(21) Application number: **87301947.5**

(22) Date of filing: **06.03.87**

The file contains technical information submitted after the application was filed and not included in this specification

(54) **Pharmaceutical mixture comprising nedocromil or a salt thereof in combination with a bronchodilator.**

(30) Priority: **15.03.86 GB 8606455**
**17.03.86 GB 8606553**

(43) Date of publication of application:
**14.10.87 Bulletin 87/42**

(45) Publication of the grant of the patent:
**30.10.91 Bulletin 91/44**

(84) Designated Contracting States:
**DE ES NL SE**

(56) References cited:
**BE-A- 902 185**

**CHEMICAL ABSTRACTS, vol. 103, 1985, page 71, abstract no. 153773z, Columbus, Ohio, US; R.P. EADY et al.: "The effect of nedocromil sodium and sodium cromoglycate on antigen-induced bronchoconstriction in the Ascaris-sensitive monkey", & BR. J. PHARMACOL. 1985, 85(2), 323(5)**

(73) Proprietor: **FISONS plc**
**Fison House Princes Street**
**Ipswich Suffolk IP1 1QH(GB)**

(72) Inventor: **Cairns, Hugh**
**2 Laneshaw avenue**
**Loughborough Leicestershire(GB)**

(74) Representative: **Craig, Christopher Bradberry et al**
**Fisons plc 12 Derby Road**
**Loughborough Leicestershire LE11 0BB(GB)**

Rank Xerox (UK) Business Services

BIOLOGICAL ABSTRACTS, 1986, abstract no. 269888; R. TOWNLEY et al.: "Effect of nedocromil sodium given by pressurized aerosol in the immediate response to bronchial antigen challenge", & J. ALLERGY CLIN. IMMUNOL. 77 (1 PART 2), 1986, 184

BIOLOGICAL ABSTRACTS, 1987, abstract no. 157595; P. DOROW: "A double-blind group comparative trial of nedocromil sodium and placebo in the managemeent of bronchial asthma in steroid-dependent patients", & EUR. J. RESP. DIS. SUPPL. 69 (147), 1986, 317-

BIOLOGICAL ABSTRACTS, 1987, abstract no. 157592; F. CUA-LIM et al.: "A double-blind comparative trial of nedocromil sodium and placebo in the management of bronchial asthma in patients routinely using oral bronchodilators", & EUR. J. RESPIR. DIS. SUPPL. 69(147), 1986, 306-31-

EP 0 241 134 B1

## Description

This invention relates to new compounds, mixtures and methods for their preparation.

Nedocromil sodium, the sodium salt of 9-ethyl-6,9-dihydro-4,6-dioxo-10-propyl-4H-pyrano[3,2-g]-quinoline-2,8-dicarboxylic acid has been recommended for use in the treatment of allergic and other conditions, in particular of reversible obstructive airway disease.

Bronchodilators and steroids have also been recommended for use in the treatment of reversible obstructive airway disease. However therapy using a combination of active ingredients, particularly fixed dose combinations, can cause problems and consequently there is a considerable prejudice against the use of such combinations. In many patients treatment with a bronchodilator on a regular basis over a prolonged period, eg six weeks, leads to a diminution in response to the therapeutic agent, leaving the patient unprotected. In some cases, prolonged administration of a bronchodilator can even lead to rebound bronchoconstriction. In other cases, one of the components of the mixture may, on its own, be sufficient to control the disease, rendering the other components superfluous.

We have now found that mixtures of nedocromil sodium with bronchodilators, salts of 9-ethyl-6,9-dihydro-4,6-dioxo-10-propyl-4H-pyrano[3,2-g]quinoline-2,8-dicarboxylic acid with bronchodilators, and mixtures of these salts with nedocromil sodium or the agent at the appropriate therapeutic ratio, possess the advantage that they are both palliative and prophylactic, are more effective, suffer less from loss of responsiveness, produce fewer side effects, reduce bronchial hyperactivity, can be used at lower doses, can be administered directly to the site of the disease, eg by inhalation, can be administered less frequently, eg 1-2 times per day, can be administered as a long term therapy, are longer acting, reduce steroid induced candidiasis, are more stable, are synergistic, cause better patient compliance, have a less offensive taste or possess other desirable properties as compared to the steroids or bronchodilators when used on their own, nedocromil sodium when used on its own or certain other mixtures when tested in relevant pharmacological models.

According to the invention there is provided a pharmaceutical mixture comprising

a) one or more of 9-ethyl-6,9-dihydro-4,6-dioxo-10-propyl-4H-pyrano[3,2-g]quinoline-2,8-dicarboxylic acid or a pharmaceutically acceptable salt thereof, as 'active ingredient A' in combination with

b) one or more of a bronchodilator or a pharmaceutically acceptable salt thereof as 'active ingredient B'.

We prefer to use the disodium salt of 9-ethyl-6,9-dihydro-10-propyl-4H-pyrano-[3,2-g]quinoline-2,8-dicarboxylic acid, which is known as nedocromil sodium. We also prefer to use only one active ingredient A in admixture with one bronchodilator as active ingredient B.

Bronchodilators that ingredient B may represent include isoprenaline. However, we prefer the bronchdilator to be $B_2$ selective.

A $B_2$-selective bronchodilator is a beta adrenoceptor agonist which has a greater affinity for $B_2$ adrenoceptors, stimulation of which results in relaxation of bronchial (and also arterial and uterine) muscle, than for $B_1$ adrenoceptors, agonists of which produce cardiac stimulation (and lipolysis). For the determination of $B_2$ selectivity, a comparison is made of the activity of the compound, (a) as a relaxant of isolated guinea pig tracheal preparations with spontaneous tone ($EC_{50}$ = molar concentration giving 50% of maximum relaxation response to isoprenaline), and (b) in increasing atrial beat frequency of isolated spontaneously beating atria from guinea-pig hearts ($EC_{50}$ = molar concentration causing 50% of maximum increase in rate induced by isoprenaline). A $B_2$ selective compound will have a lower $EC_{50}$ on the trachea than on the atria. A selectivity ratio can be determined which is the anti log of the difference between the negative log $EC_{50}$ (trachea) and negative log $EC_{50}$ (atria). The greater this selectivity ratio the more selective is the compound for $B_2$ adrenoceptors.

We prefer the $B_2$ selective bronchodilator to be long acting. By the term 'long acting' we mean that the compound has a longer duration of action as a relaxant of bronchial smooth muscle in pharmacological tests than does isoprenaline or orciprenaline. For duration of action studies, a comparison is made of the effect of equiactive i.v. doses as inhibitors of acetyl choline or histamine induced bronchospasm in anaesthetised guinea-pigs. The duration of action of isoprenaline in such a test is relatively short (control response back to normal in 10 to 25 minutes). In the case of a compound with a longer duration of action, the control response would only return to normal over a several-fold longer period of time.

Specific bronchodilators which may be mentioned are terbutaline, fenoterol, reproterol, pirbuterol, rimiterol, orciprenaline and in particular salbutamol.

According to the invention there is also provided a salt of 9-ethyl-6,9-dihydro-4,6-dioxo-10-propyl-4H-pyrano[3,2-g]quinoline-2,8-dicarboxylic acid with a basic $B_2$ selective bronchodilator.

The salt may be made by a metathetical process, eg by reacting a suitable salt, such as the disodium salt, of 9-ethyl-6,9-dihydro-4,6-dioxo-10-propyl-4H-pyrano[3,2-g] quinoline-2,8-dicarboxylic acid with an appropriate salt, eg the hydrochloride or sulphate, of the base of the agent suitable for inhalation. However the salt is preferably produced by reacting the free acid, 9-ethyl-6,9-dihydro-4,6-dioxo-propyl-4H-pyrano[3,2-g]-quinoline-2,8-dicarboxylic acid with the free base of the agent suitable for inhalation, as such a process does not produce a bi-product inorganic salt. The reaction may be carried out in a solvent which is inert under the reaction conditions. The solvent is preferably one in which the desired salt is soluble, eg water. The desired salt may be isolated and purified, for example, by crystallisation or by freeze drying.

According to the invention we therefore provide the salt when not in solution, eg the salt when in a substantially dry form, or when in admixture with insufficient liquid, eg water, to dissolve it all.

The salt may, if desired, be used in conjunction with one or more other salts of 9-ethyl-6,9-dihydro-4,6-dioxo -10-propyl-4H-pyrano[3,2-g]quinoline-2,8-dicarboxylic acid, notably the disodium salt thereof.

The ratio of active ingredients A and B in the composition will vary with the particular active ingredients, and the specific purpose for which the composition is intended. When active ingredient A is itself a salt of 9-ethyl-6,9-dihydro-10-propyl-4H-pyrano [3,2-g]quinoline-2,8-dicarboxylic acid with a basic $B_2$ selective bronchodilator, it may be necessary to use, none, or only a very small proportion of 'free' basic bronchodilator or of another salt thereof as active ingredient B.

A suitable dose of active ingredient A for inhalation is in the range 1 to 100mg and preferably 1 to 20mg, (measured as nedocromil sodium).

A suitable dosage of active ingredient B for inhalation and a suitable number of parts by weight of active ingredient A (measured as nedocromil sodium) to one part by weight of active ingredient B are specified below:

| Compound | Dose in micro g | Ratio A:B (pressurised aerosol formulation) |
|---|---|---|
| Salbutamol (as the sulphate) | 50 to 300, preferably 50 to 200 | 4 to 20 : 1 |
| Terbutaline (as the sulphate) | 100 to 600 preferably 100 to 500 | 2 to 10 : 1 |
| Fenoterol (as the hydrobromide) | 100 to 500 | 2 to 10 : 1 |
| Pirbuterol (as the hydrochloride) | 100 to 500 | 2 to 15 : 1 |
| Reproterol (as the hydrochloride) | 150 to 750 | 1.3 to 6.7 : 1 |

It is much preferred that the dose of ingredient B be such as to give a sustained rather than a transitory action. The mixture may be administered as divided doses from 1 to 6, and preferably 2 to 4, times per day. Each dose may comprise one or more unit doses.

The mixture may be administered as divided doses from 1 to 6, and preferably 2 to 4, times per day. Each dose may comprise one or more unit doses.

The specific ratios of particular active ingredients A and B in any composition according to the invention can vary over a wide range. However we prefer a composition containing from 0.4 to 400 parts by weight, and more preferably from 2 to 200 parts by weight of active ingredient A (measured as nedocromil sodium) for each part by weight of active ingredient B.

The mixtures according to the invention may be made by mixing together the various active ingredients using conventional techniques known per se.

The salts, mixtures and compositions of the invention are useful because they possess pharmacological activity in animals; in particular they are useful because they inhibit the release and/or action of pharmacological mediators which result from the in vivo combination of certain types of antibody and specific antigen, eg the combination of reaginic antibody with specific antigen (see Example 27 of GB-C-1,292,601). The salts and compositions have also been found to inhibit the degranulation of mast cells and to interfere with reflex pathways in experimental animals and man, in particular those reflexes associated with lung function. In man, both subjective and objective changes which result from the inhalation of specific antigen by sensitised subjects are inhibited by prior administration of the new salts and compositions. Thus the new salts and compositions are useful in the treatment of reversible airway obstruction and/or to prevent the secretion of excess mucous. They are thus useful for the treatment of allergic asthma, so-called 'intrinsic' asthma (in which no sensitivity to extrinsic antigen can be demonstrated), exercise etc. induced asthma, rhinitis, farmer's lung, bird fancier's disease, bronchitis, coughs (including whooping cough) and the nasal and bronchial obstructions associated with the common cold. The new salts, mixtures and compositions are also of value in the treatment of other conditions in which antigen-antibody reactions or excess mucous secretion are responsible for, or are an adjunct to, disease.

The new salts, mixtures and compositions of the invention may be administered by a wide variety of routes and may act systemically or locally. Thus the compounds may be administered by oral or nasal inhalation to the lung, directly to the nose or eye, to the buccal cavity, oesophageally or to other available surfaces of the body. The new salts or mixtures may be administered directly to the organ or part of the body showing symptoms or to a part remote from that showing symptoms.

According to the invention there is also provided a method of treating reversible obstructive airways disease which comprises administering a atherapeutically effective amount of

a) one or more of 9-ethyl-6,9-dihydro-4,6-dioxo-10-propyl-4H-pyrano[3,2-g]quinolone-2,8-dicarboxylic acid or a pharmaceutically acceptable salt thereof as 'active ingredient A' in combination with

b) a therapeutically effective amount of one or more of a $B_2$ selective bronchodilator or a pharmaceutically acceptable derivatives thereof as 'active ingredient B', simultaneously or separated in time to a patient suffering from such a disease.

The method of treatment is preferably carried out on a regular daily basis, eg 1 - 4 times per day. The method is preferably carried out over a prolonged period, eg at least 4 weeks, more preferably at least 8 weeks. The method preferably comprises simultaneously administering fixed doses of active ingredient A with fixed doses of active ingredient B.

The new salts, mixtures and compositions of the invention may be used in a variety of dosing schedules, either on their own or in conjunction with one or more other active compounds.

The new salts of the invention, and the active components of the mixtures and compositions, may be prepared in a wide variety of particle sizes. Thus for inhalation and other uses the compounds may have a mass median diameter of from 0.01 to 10 microns, preferably from 2 to 6, and most preferably from 2 to 4, microns.

According to our invention we also provide a pharmaceutical composition comprising (preferably less than 80%, and more preferably less than 50% by weight of) a salt or mixture according to the invention, in admixture with a pharmaceutically acceptable adjuvant, diluent or carrier.

Examples of suitable adjuvants, diluents or carriers are:

for tablets, capsules and dragees; microcrystalline cellulose, calcium phosphate, diatomaceous earth, a sugar such as lactose, dextrose or mannitol, talc, stearic acid, starch, sodium bicarbonate and/or gelatin;

for suppositories; natural or hardened oil or waxes; and

for inhalation compositions; coarse lactose.

For administration by inhalation the new salts or mixtures may be formulated with a compressed gas, eg nitrogen, or a liquified propellant as a pressurised aerosol composition, the composition preferably containing from 1 to 20% w/v of the active agent(s).

The new salts or mixtures may also be administered as a nebulised solution.

The invention is illustrated by the following examples.

5

Example 1

Pressurised aerosol delivering nedocromil sodium (2mg) and salbutamol sulphate (0.1mg)

| Nedocromil sodium (micronised, dried) | 1.4420% w/w |
|---|---|
| Salbutamol (midronised) | 0.0721 |
| Sorbitan trioleate | 0.5000 |
| Dichlorotetrafluoroethane | 39.1944 |
| Dichlorodifluoromethane | 58.7915 |

Cool the dichlorodifluoromethane to -55°C and disperse the sorbitan trioleate in it using a high-shearmixer. Disperse the nedocromil sodium and the butylamino)-1-4-(hydroxy-3-hydroxymethylphenyl)-ethanol sulphate in the mix and add the dichlorotetrafluoroethane, previously cooled to -55°C. Fill the suspension into cans, and crimp a metering valve of 100µl capacity onto each can.

Example 2

Pressurised aerosol delivering nedocromil sodium (2mg) and terbutaline (0.25mg).

| Nedocromil sodium, dried, micronised | 1.4286% w/w |
|---|---|
| Terbutaline (micronised) | 0.1786 |
| Sorbitan trioleate | 0.5000 |
| Trichlorofluoromethane | 24.4732 |
| Dichlorotetrafluoroethane | 24.4732 |
| Dichlorodifluoromethane | 48.9464 |

Dissolve the sorbitan trioleate in the trichlorofluoromethane and disperse the nedocromil sodium and terbutaline sulphate in the solution. Cool the solution to -55°C and add the dichlorotetrafluoroethane and dichlorodifluoromethane, cooled to -55°C. Fill the suspension into cans and crimp a metering valve of 100µl capacity onto each can.

Example 3

Pressurised aerosol delivering nedocromil sodium (2mg) and reproterol (0.5mg).

| Nedocromil sodium, dried, micronised | 1.4663% w/w |
|---|---|
| Reproterol hydrochloride (micronised) | 0.3666 |
| Sorbitan trioleate | 1.0000 |
| Trichlorofluoromethane | 10.0000 |
| Dichlorotetrafluoroethane | 13.0751 |
| Dichlorodifluoromethane | 74.0920 |

Prepare as Example 2.

The effects of mixtures, salts and compositions according to the invention were investigated in the following systems:

Example A

Rat Passive Lung Anaphylaxis Test

Female rats (Charles River) weighing 250g were passively sensitised by injecting intravenously 0.5ml of potentiated rat IgE antiserum raised against egg albumen.

The rats were anaesthetised with sodium pentobarbitone (70mg.kg) intravenously. The trachea was cannulated and the rat ventilated with a Palmer Miniature Ideal respiratory pump in a closed system. Tracheal pressure was recorded through a side arm of the cannula connected to a differential air pressure transducer (UPI, Pye Ether Ltd). The respiratory rate was set at 99 strokes per minute with a tracheal pressure of 6 cm water.

The rats were challenged, 48 hours after sensitisation, with egg albumen (25mg.kg, intravenously) and the subsequent increase in tracheal pressure expressed as a percentage of maximum possible degree of bronchconstriction ($\Delta$ PCB max), measured by clamping the trachea.

Active ingredient A and/or active ingredient B either alone or in combination, were administered intravenously in saline over the period 1 to 5 minutes before antigen challenge.

The results of giving the two compounds in combination were analysed using an analysis of variance for a factorial design with repeated measures in each cell. A square root transformation was carried out prior to the analysis.

Any antagonistic or synergistic effect of the two compounds was further examined using the method described by Berenbaum, Clin. Exp. Immunol., 28, 1 (1977). This consists of calculating the value:-

$$Z = \frac{\text{dose of A}}{Ae} + \frac{\text{dose of B}}{Be}$$

Where:

(A,B) is the combination of giving an effect X.

Ae is the dose of A alone having an effect X.

Be is the dose of B alone having an effect X.

If:

$Z < 1$, A and B are synergistic.

$Z = 1$, A and B are additive.

$Z > 1$, A and B are antagonistic.

## Example B

Inhibition of mediator release from bronchoalveolar cells

### Materials and methods

#### Materials

$[^3\text{H}]$-S-Adenosyl-L-Methionine (500mCi/mmol) and $[^{32}\text{P}]$-orthophosphate (carrier free) weere obtained from Amersham International (Amersham, Buckinghamshire, UK), sheep antiserum to rat IgE, goat antiserum to human IgE and normal goat serum from Miles Laboratories (Slough, Buckinghamshire, UK). The goat anti-human IgE and control serum were precipitated with 40% saturated ammonium sulphate (Largman et al, Methods Enzymol (1981) 74:22) and, after dialysis, the globul fraction adjusted to the original serum volume. The ascaris antigen was prepared from adult Ascaris suum pseudocoelomic fluid by chromatography on Sephadex G200 (Pharmacia, Milton Keynes, Buckinghamshire, UK), using the method of Ambler et al, J. Immunol. Meth. (1972), 1,317.

#### Infection and bronchoalveolar lavage of macaques

A group of 20 Macaca arctoides monkeys, weight 8-18kg, was used in these studies. The animals were infected with A. suum and lavaged as described in Pritchard et al, Clin. Exp. Immunol., (1983), 54:469. The lavage fluid was recovered into heparinised tubes and placed on ice.

#### Treatment of BAL cells

The lavage fluid was filtered through a 175um nylon mesh and the cells recovered by centrifugation (450g for 5 minutes at 4°C). The cells were washed and resuspended to the required working dilution in buffer (10mm HEPES-buffered Tyrode, pH 7.4 containing 1mg/ml gelatin and 5 units/ml heparin). Differential cell counts were carried out in wet preparations in Kimura's stain (20). When only histamine release was to be measured, the cells were challenged in triplicate by adding 0.05ml cells, at a density of $10^5$ mast

cells/ml, ingredient A, eg to 0.05ml buffer containing releasing agent and ingredient B, eg $(3x10^{-8}-10^{-6}M)$ and nedocromil sodium $(3x10^{-6}-10^{-4}V)$ alone and in combination at 37°C. After incubating for 20 minutes at 37°C, the release process was quenched by the addition of 0.25ml ice cold $Ca^{2+}$-,$Mg^{2+}$-free buffer containing 2mM EDTA. After centrifugation (450g for 5 minutes at 4°C), 0.2ml supernatant was sampled for histamine analysis. In all inhibiting experiments a submaximal level of challenge was chosen which, from the experience of previous lavages of that animal, released 15-25% of the total histamine, but not more than half the maximum release attainable.

Measurement of Histamine

Histamine was measured by the double isotope method of Beaven et al, Clin. Chim. Acta, (1972), 37,97 modified as described in Pritchard et al.

Example C

Non-specific Bronchial Hyperactivity in Adult Asthmatics

Adult asthmatics gave informed consent and entered the study.

Histamine challenge was performed by the method of Cockcroft, Clin. Allergy, (1977), 7, 235. Doubling concentrations of histamine acid phosphate (0.03 - 8mg/ml) were inhaled for two minutes at five minute intervals. $FEV_1$ was measured on a Vitalograph dry wedge spirometerm before and at 0.5 minutes and 1.5 minutes after each inhalation. The challenge was discontinued when $FEV_1$ had fallen by 20% or more from the baseline measure. Log-dose response curves wree constructed and the $PC_{20}$ measured by interpolation of the last two points.

Example D

Human volunteers suffering from specific allergic asthma were selected for the study. In these human volunteers an asthma attack normally follows the inhalation of an antigen to which he is specifically sensitive. The degree of asthma provoked by this method can be measured by repeated examination of the airway resistance.

A suitable designated spirometer is used to measure the forced expirataory volume at one second $(FEV_1)$ hence the changes in airway resistance.

Drug induced changes in $FEV_1$ are measured at 5 minutes after administration of the drug.

Six hours after drug administration a standard antigen challenge is administered to the human volunteer and the fall in $FEV_1$ measured at 5 minutes after the antigen administration.

## Claims

1. A pharmaceutical mixture comprising
    (a) one or more of 9-ethyl-6,9-dihydro-4,6-dioxo-10-propyl-4H-pyrano[3,2-g]quinoline-2,8-dicarboxylic acid or a pharmaceutically acceptable salt thereof as 'active ingredient A' in combination with
    (b) one or more of a bronchodilator or a pharmaceutically acceptable salt thereof as 'active ingredient B'.

2. A mixture according to Claim 1, wherein active ingredient A is nedocromil sodium.

3. A mixture according to Claim 1, comprising from 0.4 to 400 parts by weight of active ingredient A, measured as nedocromil sodium, for each part by weight of active ingredient B.

4. A mixture according to any one of the preceding Claims, wherein active ingredient B is a B-2 selective bronchodilator or a pharmaceutically acceptable salt thereof.

5. A mixture according to any one of the preceding Claims, wherein active ingredient B is salbutamol or a pharmaceutically acceptable salt thereof.

6. A mixture according to any one of Claims 1 to 3, wherein active ingredient B is terbutaline, fenoterol, reproterol, pirbuterol, rimiterol or a pharmaceutically acceptable salt thereof.

7. A salt of 9-ethyl-6,9-dihydro-4,6-dioxo-10-propyl-4H-pyrano[3,2g]quinoline-2,8-dicarboxylic acid with a basic B-2 selective bronchodilator.

8. A pharmaceutical composition comprising a mixture according to any one of the Claims 1 to 6 or a salt according to Claim 7 in admixture with a pharmaceutically acceptable adjuvant, diluent or carrier and in a form suitable for inhalation.

9. A composition according to Claim 8 in the form of a pressurised aerosol composition containing from 1 to 20% w/w of the active agent(s) and comprising from 0.05 to 1.5% by weight of a surface active agent.

## Revendications

1. Mélange pharmaceutique comprenant :
   (a) un ou plusieurs d'entre l'acide 9-éthyl-6,9-dihydro-4,6-dioxo-10-propyl-4H-pyrano[3,2-g]-quinoléine-2,8-dicarboxylique et un sel pharmaceutiquement acceptable de celui-ci comme "constituant actif A" en combinaison avec
   (b) un ou plusieurs d'entre un bronchodilatateur et un sel pharmaceutiquement acceptable de celui-ci comme "constituant actif B".

2. Mélange suivant la revendication 1, dans lequel le constituant actif A est le nédocromil sodique.

3. Mélange suivant la revendication 1, comprenant 0,4 à 400 parties en poids de constitutif actif A, mesuré en nédocromil sodique, par partie en poids de constituant actif B.

4. Mélange suivant l'une quelconque des revendications précédentes, dans lequel le constituant actif B est un bronchodilatateur β-2 sélectif ou un sel pharmaceutiquement acceptable de celui-ci.

5. Mélange suivant l'une quelconque des revendications précédentes, dans lequel le constituant actif B est le salbutamol ou un sel pharmaceutiquement acceptable de celui-ci.

6. Mélange suivant l'une quelconque des revendications 1 à 3, dans lequel le constitutif actif B est la terbutaline, le fénotérol, le reprotérol, le pirbutérol, le rimitérol ou un sel pharmaceutiquement acceptable de l'un de ceux-ci.

7. Sel de l'acide 9-éthyl-6,9-dihydro-4,6-dioxo-10-propyl-4H-pyrano[3,2-g]quinoléine-2,8-dicarboxylique avec un bronchodilatateur β-2 sélectif basique.

8. Composition pharmaceutique comprenant un mélange suivant l'une quelconque des revendications 1 à 6, ou un sel suivant la revendication 7, en mélange avec un adjuvant, diluant ou excipient pharmaceutiquement acceptable et sous une forme propre à l'inhalation.

9. Composition suivant la revendication 8, sous la forme d'une composition en aérosol sous pression contenant 1 à 20% p/p du ou des constituants actifs et comprenant 0,05 à 1,5% en poids d'un agent tensioactif.

## Patentansprüche

1. Pharmazeutisches Gemisch umfassend
   (a) ein(e) oder mehrere 9-Ethyl-6,9-dihydro-4,6-dioxo-10-propyl-4H-pyrano[3,2-g]chinolin-2,8-dicarbonsäure, oder ein pharmazeutisch annehmbares Salz hievon, als "aktiven Inhaltsstoff A" in Verbindung mit
   (b) einem oder mehreren Bronchodilator(en), oder einem pharmazeutisch annehmbaren Salz hievon, als "aktiven Inhaltsstoff B".

2. Gemisch gemäß Anspruch 1, worin der aktive Inhaltsstoff A Natriumnedocromil ist.

3. Gemisch gemäß Anspruch 1, umfassend 0,4 bis 400 Gewichtsteile aktiven Inhaltsstoff A, gemessen als Natriumnedocromil, pro Gewichtsteil aktiven Inhaltsstoff B.

4. Gemisch gemäß einem der vorhergehenden Ansprüchen, worin der aktive Inhaltsstoff B ein B-2-selektiver Bronchodilator oder ein pharmazeutisch annehmbares Salz hievon ist.

5. Gemisch gemäß einem der vorhergehenden Ansprüchen, worin der aktive Inhaltsstoff B Salbutamol oder ein pharmazeutisch annehmbares Salz hievon ist.

6. Gemisch gemäß einem der vorhergehenden Ansprüchen, worin der aktive Inhaltsstoff B Terbutalin, Fenoterol, Reproterol, Pirbuterol, Rimiterol oder ein pharmazeutisch annehmbares Salz hievon ist.

7. Salz von 9-Ethyl-6,9-dihydro-4,6-dioxo-10-propyl-4H-pyrano[3,2-g]chinolin-2,8-dicarbonsäure mit einem basischen B-2-selektiven Bronchodilator.

8. Pharmazeutische Zusammensetzung, umfassend ein Gemisch gemäß einem der Ansprüche 1 bis 6 oder ein Salz gemäß Anspruch 7 in Mischung mit einem pharmazeutisch annehmbaren Hilfs-, Verdünnungsmittel oder Träger und in einer zur Inhalation geeigneten Form.

9. Zusammensetzung gemäß Anspruch 8 in Form einer Aerosolzusammensetzung unter Druck, die 1 bis 20 % G/G des (der) aktiven Inhaltsstoffs(stoffe) enthält und 0,05 bis 1,5 Gew.% eines oberflächenaktiven Mittels umfaßt.